# EUROPEAN PATENT SPECIFICATION

(11) **EP 3 129 498 B1**
(45) Date of publication and mention of the grant of the patent: **12.12.2018**
(21) Application number: 15719064.6
(22) Date of filing: 10.04.2015
(51) Int. Cl.: C12Q 1/68, C12N 15/10

(54) **NUCLEIC ACID PURIFICATION METHOD**
VERFAHREN ZUR REINIGUNG VON NUKLEINSÄURE
PROCÉDÉ DE PURIFICATION D'ACIDES NUCLÉIQUES

(30) Priority: 11.04.2014 US 201461978322 P
(43) Date of publication of application: 15.02.2017
(73) Proprietor: FUJIFILM Wako Pure Chemical Corporation, Osaka-shi, Osaka 540-8605 (JP)
(72) Inventor: HAMADA, Takatoshi, Amagasaki-shi Hyogo, 661-0963 (JP); SWENSON, David, Mountain View, CA 94043 (US)
(74) Representative: Winter, Brandl, Fürniss, Hübner, Röss, Kaiser, Polte - Partnerschaft mbB
(86) International application number: PCT/US2015/025335
(87) International publication number: WO 2015/157650

(56) References cited:
- WO-A1-01/62976
- WO-A1-95/06652
- WO-A1-2010/014970
- WO-A1-2011/017251
- WO-A1-2012/028737
- WO-A1-2013/083260
- WO-A2-2012/155072
- US-B1- 7 067 287
- SCOTT M. BERRY ET AL: "One-step purification of nucleic acid for gene expression analysis via Immiscible Filtration Assisted by Surface Tension (IFAST)", LAB ON A CHIP, vol. 11, no. 10, 1 January 2011 (2011-01-01), page 1747, XP055061328, ISSN: 1473-0197, DOI: 10.1039/c1lc00004g
- SUR KUNAL ET AL: "Immiscible Phase Nucleic Acid Purification Eliminates PCR Inhibitors with a Single Pass of Paramagnetic Particles through a Hydrophobic Liquid", JOURNAL OF MOLECULAR DIAGNOSTICS,THE, AMERICAN SOCIETY FOR INVESTIGATIVE PATHOLOGY, US, vol. 12, no. 5, 1 September 2010 (2010-09-01), pages 620-628, XP009169239, ISSN: 1525-1578

## Description

### Field of the Invention

The invention relates to methods for purifying nucleic acids such as DNA or RNA, including purifying such nucleic acids from clinical samples or other blood, cell, or tissue sample types.

### Background of the Invention

Molecular biology techniques employing DNA and RNA samples often rely on obtaining purified samples of the nucleic acids. There are numerous sources of DNA or RNA, such as tissue, cells, whole blood, plasma, serum, extracts, which may be obtained from an organism (e.g., human, animal, plant, bacteria, virus, etc.), as well as tissue or cell cultures developed or maintained in a lab. Further use of the DNA or RNA generally requires isolating the DNA or RNA from the other components in the sample source, so that the polynucleic acids are free of other cellular enzymes, proteins, compounds, and/or debris. Isolating the DNA or RNA from these other materials enables subsequent processing of the polynucleic acids, by, for example, PCR, RT-PCR, TMA, LAMP, LCR, sequencing, nucleic acid hybridization analysis, or any of the numerous techniques known in the art based on enzymatic reactions or hybridization reactions using nucleic acids.

One of the earliest techniques for isolating DNA or RNA was to lyse a cell or tissue sample using, for example, proteinase K, add an equal volume of a phenol/chloroform solution to the lysate to extract non-nucleic acid materials (e.g., proteins, organelles, etc.), and remove the aqueous phase containing the nucleic acids for further use. Usually, the nucleic acids are recovered from the aqueous phase by precipitation with ethanol or isopropanol. Modifications of the technique include further adding to the organic phase a small amount of isoamyl alcohol to aid in deactivating RNases, and/or adding a chaotropic agent such as guanidinium thiocyanate to aid in protein denaturation and enzyme inactivation. Although phenol/chloroform extraction is considered to be the "gold standard" for purifying nucleic acids by removing non-nucleic acid materials because of the high purity and high recovery it provides, there are drawbacks to the method, such as being laborious, difficult to automate, and requiring the use of hazardous organic solvents.

Subsequently, techniques based on absorbing the nucleic acids to a solid phase, washing away unwanted materials, and eluting the nucleic acids have been developed as disclosed in WO 2012/028737 A1, US 7067287 B1, WO 95/06652 A1, WO 01/62976 A1. The solid phase is often silica particles, but other materials such as glass fibers, beads, or powder, hydroxyapatite, anionic exchange resins, and diatoms are also used. Polynucleic acids are bound to the solid phase in the presence of chaotropic salts, non-bound materials are washed away using alcohol-containing solutions, and finally the polynucleic acids are eluted from the solid phase using a low ionic strength solution to obtain the polynucleic acids in substantially pure form.

Several companies offer kits for purifying DNA and RNA that feature use of a solid phase material. The kits generally provide the solid phase material in the form of a column, a membrane, or as a coating on paramagnetic particles. When paramagnetic particles are used, a magnet is used to sequester the particles while wash or elution buffers are removed as described by Scott M. Berry et al. ("One-step purification of nucleic acid for gene expression analysis via Immiscible Filtration Assisted by Surface Tension (IFAST)", Lab Chip, 2011,11, 1747-1753) as well as by Sur Kunal et al. ("Immiscible Phase Nucleic Acid Purification Eliminates PCR Inhibitors with a Single Pass of Paramagnetic Particles through a Hydrophobic Liquid", J Mol Diagn. 2010 Sep; 12(5): 620-628). However, paramagnetic particles are relatively expensive, and manipulating the particles increases the costs of automation. When columns or membranes are used as the solid phase, the wash and elution buffers are drawn through by either centrifugation or applying a vacuum. The columns and membranes need to be designed and processed such that the wash solutions are effective at passing through the entire void volume. Liquid hold-up within the solid phase, or similarly, inadequate washing out of the void space in the solid phase means that impurities, inhibitors, or other compounds that will interfere with or contaminate the nucleic acid product will not be removed. Centrifugation is often used to attempt to uniformly remove the liquid, but automated systems are necessarily larger, more complex, and costly. Generally, methods that have tried to improve upon the art still suffer from one or more of the following problems: (1) low yields of nucleic acid product, (2) increased volumes of wash solution waste; (3) nucleic acid product solutions that are more dilute than is desirable; (4) PCR inhibitors remain; and (5) lengthy processing time.

Thus, a method based on simple liquid-handling steps that are easy to automate is still desired.

The inventors observed that the use of glass microfiber filters as a solid phase material for nucleic acid purification does not yield a sufficiently pure product for use in subsequent enzymatic-based processing using standard protocols. For example, a sample of cells was treated with a lysis buffer and the lysate was passed through the glass microfiber filter by pressure to bind the nucleic acids (DNA and RNA) to the glass fibers. The filter was then washed twice with aqueous ethanol by pressure, and the nucleic acid was eluted using nuclease-free water. Subsequent attempts to conduct PCR using the obtained material as a target resulted in failed amplification reactions. The presence of inhibitors in the obtained material was confirmed through the use of control reactions.

Accordingly, there remains a need for nucleic acid purification methods that are compatible with thick and/or larger and/or varied pore size filters in order to achieve rapid, high throughput, high purity yields of nucleic acid samples, as well as methods that are amenable to automation in a compact system, yet that are processing sample volumes of about 1 mL or more while decreasing operating costs.

### Summary of the Invention

Methods for purifying polynucleic acids employing filters as a solid phase and pressure-driven operation are provided. A first embodiment comprises (1) adsorbing polynucleic acids to a filter, (2) washing the filter with an immiscible fluid wash solution, and (3) eluting the polynucleic acids from the filter using (i) an aqueous-based elution solution and (ii) an immiscible fluid push solution.

A second embodiment comprises after step (1) and before step (2), washing the filter with an alcohol-based wash solution.

A third embodiment comprises after step (1) and before step (2), washing the filter with an immiscible fluid wash solution and then washing the filter with an alcohol-based wash solution.

Further embodiments include washing the filter with two or more different compositions of alcohol-based wash solutions, and/or two or more different compositions of immiscible fluid wash solutions.

In another embodiment, the elution step is performed by placing the aqueous-based elution solution at the entrance side of the filter, layering an immiscible fluid wash solution on top of or behind the aqueous-based elution solution, and passing the two solutions through the filter to obtain a solution of purified polynucleic acids in the aqueous phase.

The methods of the various embodiments can be used to purify any type of polynucleic acid, such as DNA, RNA, and/or mixtures of DNA and RNA. The polynucleic acids may be of natural origin (for example, extracted from any type of cell) or synthetic.

A system is also provided that comprises a solid phase and a container with a porous bottom that can retain the solid phase within the container, liquid delivery means for delivering at least one immiscible wash solution, an aqueous-based elution solution and an immiscible fluid push solution, to the solid phase in the container, which can perform the methods of the invention. The system may further comprise a pressure source that can provide a pressure head to drive any of the solutions through the solid phase in the container. The system may also further comprise a vacuum source that can induce the flow of any of the solutions through the solid phase in the container. In yet another embodiment, the system may further comprise a centrifuge capable of providing centrifugal force to drive any of the solutions through the solid phase in the container.

In some embodiments, the apparatus for performing the method has a compartment and filter for processing a single sample, while in other embodiments multiple compartment and filter sets are provided for multi-sample processing, which in some cases can be carried out in parallel. As an example of multi-sample processing, multiwell microtitre plates with porous well-bottoms can be used as a container, and a filter can be fitted in each well. An apparatus for single-sample or multi-sample processing can be incorporated into system for performing the methods either manually, semi-automatically, or automatically.

These and other objects and features of the invention will become more fully apparent when the following detailed description of the invention is read in conjunction with the accompanying drawings.

### Brief Description of the Drawings

Fig. 1 shows one embodiment of an apparatus useful for performing embodiments of the purification method.
Fig. 2A-2B shows the growth curve for the real-time RT-PCR analysis of the purified nucleic acids samples obtained according to an embodiment of the invention described in Example 3 (reference).
Fig. 3A-3B shows the melt curve for the product of the real-time RT-PCR analysis of the purified nucleic acids samples obtained according to an embodiment of the invention described in Example 3 (reference).
Fig. 4A-4B shows the results of an electrophoretic analysis of the PCR product obtained from the samples prepared according to an embodiment of the invention described in Example 3 (reference).
Fig. 5 shows the growth curve for the real-time PCR analysis of the purified nucleic acids samples obtained according to an embodiment of the invention described in Example 5 (reference).
Fig. 6 shows the melt curve for the product of the real-time PCR analysis of the purified nucleic acids samples obtained according to an embodiment of the invention described in Example 5 (reference).
Fig. 7 shows the results of an electrophoretic analysis of the PCR product obtained from the samples prepared according to an embodiment of the invention described in Example 5 (reference).

### Detailed Description

The DNA or RNA of interest includes the DNA or RNA of all organisms, such as humans, other mammals, other animals, plant, bacteria, viruses, or any other life form, living or deceased. The DNA or RNA in some embodiments are naturally occurring polynucleic acids, and the methods may be used as part of a protocol for extracting the polynucleic acids from their natural source inside a cell, or for purifying the polynucleic acids from a lysate. The source may be a clinical sample, and the polynucleic acids are being analyzed as part of a diagnostic assay. Naturally occurring polynucleic acids may be derived from any type of sample, such as a cell culture, a tissue sample, a blood sample, and the like. The DNA or RNA in other embodiments may be the product of a molecular biological reaction. For example, in some embodiments a DNA sample may be derived from a natural DNA source, such as by amplifying genomic DNA, or a natural RNA source, such as cDNA produced from an RNA transcription product.

The DNA or RNA polynucleic acids can be of any length, and the method can be applied to a combination of lengths. Typically, the nucleic acids may be as small as 40 bases, and may be as long as about 50 kilobases. The nucleic acids may be single-stranded or double-stranded, or any other multistranded form. In some embodiments, both DNA and RNA together may be the subject of the purification method. In other embodiments, the wash and elution conditions are optimized for the purification of either DNA or RNA.

In the methods, the DNA and/or RNA are adsorbed onto a solid phase, other materials are washed away, and then the DNA and/or RNA are eluted off the solid phase. To wash away the impurities from the absorbed nucleic acids, the solid phase is washed with an immiscible fluid wash solution as described herein. The wash with an immiscible fluid may be performed one or two or more times, and other washes may also be performed, as described below. Multiple washes with an immiscible fluid may be performed consecutively and/or washes with other solutions may intervene.

The solid phase may also be referred to as a filter. In some embodiments, the solid phase is a glass-based material, such as borosilicate glass. In some embodiments the borosilicate glass filter is binder-free. For example, the filter may be a borosilicate glass binder-free microfiber, which may be in the form of a filter of any shape. The thickness of the filter may be in the range of 50 to 3000 µm, and exemplary embodiments include 100 µm, 250 µm, 500 µm, 675 µm, 1000 µm, or 2000 µm. The particular thickness may depend on the manufacturer. Furthermore, filters can be stacked to increase the total thickness of the solid phase. Stacking filters can be used to, for example, increase the binding capacity of the device. The filter of microfibers may be formed such that the structure provides for the retention of particles in the range of 0.5 to 3.0 µm, and exemplary embodiments include sizes of 0.7 µm, 1.0 µm, 1.5 µm, 2.0 µm, or 2.7 µm. Again, the particular particle size retained may depend on the manufacturer. A filter may be multilayered, containing more than one pore size, or, when more than one filter is stacked in a container the individual filters may have the same or different pore sizes. In one embodiment, the filter has the properties of a Whatman glass microfiber binder free filter, grade GF/B (675 µm thick, 1.0 µm particle retention rating). In other embodiments, the filter has the properties of a Whatman microfiber filter, grade GF/A (260 µm thick, 1.6 µm particle retention rating), GF/C (260 µm thick, 1.2 µm particle retention rating), GF/D (675 µm thick, 2.7 µm particle retention rating), or GF/F (420 µm thick, 0.7 µm particle retention rating).

Immiscible fluid wash solution. An immiscible fluid wash solution is used to wash the filter, at least once, after the nucleic acid material has been adsorbed to the filter. The immiscible fluid wash solution comprises a fluid that, separate and apart from when practicing the methods of invention, (i) when contacted with water forms a substantially separate phase from the water, and in preferred embodiments forms a meniscus between the fluid and the water. In preferred embodiments, the fluid also (ii) has a specific gravity less than that of water, (iii) does not substantially interfere with or inhibit enzymatic reactions involving the nucleic acid that is the object of the purification method, and (iv) is chemically compatible with the purification system apparatus components, such as the filter and the container housing the filter.

The immiscible fluid wash solution, when positioned at the entrance side of the filter and then moved through the filter, functions to push through the void space of the filter, in the direction of the flow, the solution that occupied the void space. In particular, the immiscible fluid wash solution functions to push through any aqueous solution or alcohol-based wash solution that occupied the void space in the filter. In another aspect, the immiscible fluid wash solution functions to remove or substantially reduce the amount of enzyme inhibitors held up in the filter which would otherwise be eluted with the nucleic acid materials. In another aspect, the immiscible fluid wash solution functions to remove or substantially reduce the amount of components from samples that might interfere with methods for detecting the nucleic acid materials. For example, bilirubin in, e.g., serum samples, can be removed or reduced by washing with a immiscible fluid wash solution.

In some embodiments, the immiscible fluid is a hydrophobic polymer. The polymer may be an inorganic polymer, and a preferred embodiment is silicone oil (also known as silicone fluid). In some embodiments, the polymer may be an organic polymer, such mineral oil, paraffin oil, Vapor Lock (Qiagen Inc., Valencia, CA), baby oil, or white oil. The polymer may be a natural, synthetic, or semi-synthetic product. Other examples include fish oils or vegetable oils derived from, e.g., soybean, olive, peanut, corn, or canola. The immiscible fluid wash solution may comprise more than one hydrophobic polymer type. In some embodiments, the immiscible fluid is a non-polymeric organic compound. In some embodiments, the immiscible fluid wash solution may comprise a polymer and a non-polymeric organic compound. In some preferred embodiments, the immiscible fluid has a viscosity in the range of 1 to 20 cSt, and in some preferred embodiments the viscosity is in the range of 1 to 10 cSt.

In addition to meeting the four criteria (i)-(iv) listed above for the immiscible fluid, a non-polymeric compound should also be physically compatible with any downstream processing involving the purified nucleic acids. For example, if the nucleic acids are to be incubated at or heated to a high temperature, then if the immiscible fluid remains present in the sample, it should have a vapor pressure and boiling point that does not interfere with the process. That is, the boiling point should be suitably greater than the process temperature, and/or the vapor pressure should be suitably low at the process temperature such that volatility or volume expansion does not interfere with the process. For example, if the purified nucleic acids are to be used in a thermocycled reaction having a denaturing step at e.g., 95°C, then the boiling point of any immiscible fluid should be sufficiently greater than this temperature.

The specific gravity of the immiscible fluid is generally less than that of water. Accordingly, the wash solution will tend to remain above any aqueous solution within the filter when the immiscible fluid wash solution is placed on the filter during the performance of the method. In this way, the immiscible fluid wash solution can be moved through the filter material and push through the aqueous solution that had been held up in the voids in the filter.

One function of the immiscible fluid wash solution is to reduce or remove substances that interfere with or inhibit enzymatic reactions. In some embodiments of the purification method, some of the immiscible fluid wash solution may remain in the eluted product. When immiscible fluid wash solution remains in the product, the eluate and the immiscible fluid may be used together in a subsequent reaction, where the immiscible fluid can, by virtue of having a lower specific gravity than water, rise to the upper boundary of the sample. In one embodiment, the immiscible fluid may function as a vapor barrier.

In some embodiments of the purification method, the immiscible fluid wash solution may be prevented from being collected with the eluted product. In others, the immiscible fluid wash solution may be removed from the eluted product by separating the phases, extraction, and other chemical or physical techniques. Whether the immiscible fluid wash solution remains in the eluted product or not, the immiscible fluid wash solution itself should also be considered for its compatibility with any downstream enzymatic reactions.

Candidate immiscible fluids are available in numerous grades and similar polymers can be prepared from various sources, thus the compatibility of the immiscible fluid and the immiscible fluid wash solution with any intended downstream reactions should be confirmed when selecting the fluid or formulating the solution. For example, if the purified nucleic acid material is to be used in a PCR reaction, then the fluid and the wash solution can be directly added to a sample and the inhibitory effect, if any, can be determined.

Those of skill in the art are familiar with the need for and methods for confirming the compatibility of a reagent in an enzymatic reaction. Immiscible fluids and immiscible fluid wash solutions suitable for use in the purification methods described herein are those that do not substantially interfere with or inhibit downstream reactions, including enzymatic reactions, using the purified product. For example, preferred wash solution fluids do not interfere with or substantially inhibit nucleic acid amplification reactions. Exemplary amplification reactions include PCR, RT-PCR, TMA, LAMP, LCR, and the like. To interfere with or substantially inhibit a reaction means that the amount and type of product produced is different from the amount and type of product produced in the absence of the immiscible fluid(s) or the immiscible fluid wash solution. It is not necessary that the immiscible fluid(s) or immiscible fluid wash solution have no effect on the subsequent reaction, just that any effect that it may have be within a tolerable level for the intended application.

The immiscible fluid and wash solution should also be chemically compatible with the apparatus used to perform the purification methods. For example, immiscible fluids and wash solutions should not dissolve, leach, or deform apparatus components. The apparatus includes at least a filter and a container housing the filter.

Alcohol-based wash solution. In some embodiments, an alcohol-based wash solution is used to wash the solid phase. In some embodiments, two or more types of alcohol-based wash solution are used. The alcohol-based wash solution comprises a lower alkanol, such as a C1-C4 alkanol. The alkyl group may be a straight or branched chain, and may contain one or more hydroxyl groups. Examples include methanol, ethanol, propanol, isopropanol, butanol, isobutanol, and tert-butanol. Further examples include ethylene glycol, propylene glycol, glycerol, and other polyols. In preferred embodiments, the alcohol is ethanol. More than one type of alcohol may be present in the wash solution.

The alcohol-based wash solution may comprise about 5% up to 100% alcohol. In some preferred embodiments the alcohol-based wash solution comprises 10 to 70% alcohol, and in other preferred embodiments, 20 to 50% alcohol. When the wash solution is not 100% alcohol, another liquid component may be water. In some embodiments, the alcohol-based wash solution is 24% ethanol in water, or 50% ethanol in water, or 70% ethanol in water, or 100% ethanol. In some embodiments, the solution may further contain one or more salts, buffers, nonionic and/or ionic surfactants, hydrophilic polymers, preservatives, and/or antimicrobial agents. Any such component may be included as long as it does not cause substantial loss of adsorbed nucleic acids from the solid phase or interfere with any downstream processing of the nucleic acids once they are eluted from the solid phase. Generally, any such components should be compatible with nucleic acids and the further downstream use, including enzymatic reactions targeting the nucleic acids.

In this wash solution, as well as in the other wash solutions and other reagents used when processing the nucleic acids, the water used in the solution is generally distilled water, and is generally DNase and/or RNase-free, according to the needs and purpose of performing the method.

Preferred examples of salts include sodium chloride, potassium chloride, and sodium, potassium, or ammonium acetate, and preferred examples of buffers include Tris-HCI, HEPES, and other Good's buffers. The salt concentration is typically 10 to 200 mM. The buffer concentration is typically 10 to 500 mM, preferably 20 to 200 mM, and the pH is 6 to 11, preferably 7 to 10. Preferred examples of nonionic surfactants include polysorbate 80 (one trade name is Tween 80®), other polysorbates, Nonidet P-40®, Triton X-100®, and the like. Nonionic surfactants are typically present in a concentration of 1 to 10 %, preferably 1 to 5 %. Preferred examples of hydrophilic polymers include polyethylene glycol, such as PEG 8000. Preferred examples of preservatives include sodium azide, ProClin 150, ProClin 200, and ProClin 300. Preservatives are typically present in a concentration of 0.1 to 10%. These examples of salts, buffers, surfactants, and preservatives are not meant to be exclusive, but representative, and substitutes can be readily found by one of skill in the art.

In some embodiments, the alcohol-based wash solution is 50% ethanol in 100 mM NaCl aqueous solution, or 50% ethanol in 10 mM Tris-HCI, 100 mM NaCl aqueous solution, or 50% ethanol in 10 mM Tris-HCI aqueous solution. In some embodiments, the alcohol-based wash solution is 24% ethanol in a pH 7.5 buffered aqueous solution, or 24% ethanol in a 25 mM HEPES (pH 7.5) aqueous solution, or 24% ethanol in a 25 mM HEPES (pH 7.5), 30 mM NaCl aqueous solution. In some embodiments, the alcohol-based wash solution further comprises 8.5% (w/v) PEG 8000, and/or further comprises 0.1% (v/v) ProClin 300. These examples are not meant to be exclusive, but merely exemplary of the types of alcohol-based wash solutions that can be used. One of skill in the art would appreciate that many solutions, varying in the choice of components, concentrations, pH, and the like, described herein can be used.

Elution solution. An aqueous-based elution solution is used to elute the adsorbed nucleic acids from the solid phase. The elution solution comprises an aqueous solution, and may be up to 100% water. In some embodiments, the elution solution further contains a salt and/or a buffer. Preferred examples of salts include sodium chloride, potassium chloride, and sodium, potassium, or ammonium acetate, and preferred examples of buffers include Tris-HCI, HEPES, and other Good's buffers. The salt concentration is typically up to 50 mM. The buffer concentration is typically 10 to 500 mM, preferably 20 to 200 mM, and the pH is 6 to 11, preferably 7 to 10. These examples of salts and buffers are not meant to be exclusive, but representative, and substitutes can be readily found by one of skill in the art. The water used in the elution solution is generally distilled water, and is generally DNase and/or RNase-free, according to the needs and purpose of performing the method. In one embodiment, an elution solution for eluting DNA is 10 mM (pH 9.0) Tris-HCI. In one embodiment, an elution solution for eluting RNA is distilled water. In some embodiments, low ionic strength, for example, less than 50 mM, aqueous solutions are used. Persons of ordinary skill in the art can test the suitability of an elution solution by assessing the yield of nucleic acid material released from the solid phase. Generally, the yield of material released from the solid phase is high, and may be at least 80%, or at least 90%, or may be nearly quantitative. Lower yields may be acceptable, as long as the amount released is suitable for the goals of the application.

Immiscible fluid push solution. An immiscible fluid push solution is used to assist moving the elution solution through the filter. The immiscible fluid push solution comprises a fluid that, separate and apart from when practicing the methods of the invention, (i) when contacted with water forms a substantially separate phase from the water, and in preferred embodiments forms a meniscus between the fluid and the water. In preferred embodiments, the fluid also (ii) has specific gravity less than that of water, (iii) does not substantially interfere with or inhibit enzymatic reactions involving the nucleic acid that is the object of the purification method, and (iv) is chemically compatible with the purification system apparatus components, such as the filter and the container housing the filter.

The immiscible fluid push solution, when positioned at the entrance side of the filter and then moved through the filter, functions to push through the void space of the filter, in the direction of the flow, the aqueous-based elution solution that occupied the void space. The elution solution can be collected efficiently by using an immiscible fluid push solution in the elution step, as described below.

The immiscible fluid push solution may be a hydrophobic polymer or a non-polymeric organic compound, or combinations thereof, as described above for the immiscible fluid wash solution. In some preferred embodiments, the immiscible fluid has a viscosity in the range of 1 to 300 cSt, in some preferred embodiments the range is 10 to 100 cSt, and in other preferred embodiments the range is 20 to 50 cSt. The other properties and considerations for selecting an immiscible fluid push solution are the same as those described above for the immiscible fluid wash solution.

In the purification methods described herein, a nucleic acid material is adsorbed onto a solid phase, other materials are washed away using a water-immiscible-fluid-based wash solution, and then the nucleic acid material is eluted off the solid phase.

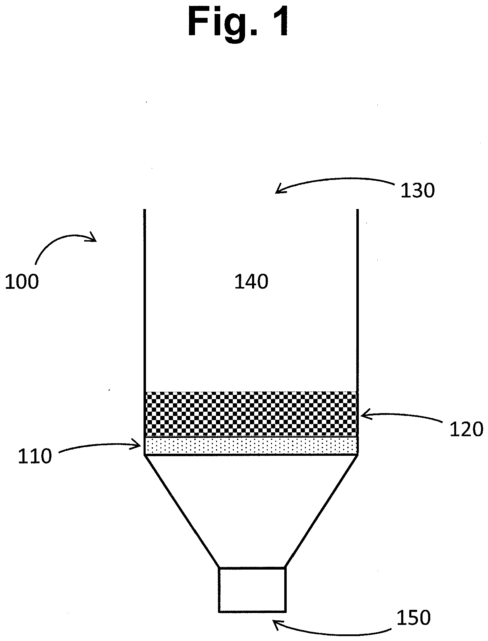

The solid phase, generally a filter, is fitted in a container configured such that solution added to one compartment in the container can be made to pass through the filter. The container may be circular, square, or polyhedral in cross-section. The container may comprise a stand-alone unit or part of a closed or partially-closed system in which materials are delivered directly to the entrance side of the filter by means of tubing or other delivery means. An example of a stand-alone embodiment is shown in Fig. 1. A container 100 is provided having a first opening 130 and a second opening 150, and a porous support 110 spanning the cross-section of the container. A first compartment 140 in container 100 exists in the space defined by the container walls, the porous support 110, and the first opening 130. For convenience, the volume of first compartment 140 can configured to accommodate the typical volume of a wash solution and an elution solution to be used in the method. Nonetheless, serial applications of the nucleic acid material and serial washes are also contemplated. The compartment does not have to be sized to accommodate the largest volume solution that will be used. In some embodiments, first compartment 140 can accommodate 50 µL to 1000 µL or more of solution, or may accommodate at least up to 200 µL, or at least up to 400 µL, or at least up to 600 µL, or at least up to 800 µL of solution. A typical volume of first compartment 140 that is convenient for processing an *in vitro* diagnostic assay sample may be in the range of 50-1000 µL or more. In other embodiments, the container can be configured for a continuous flow of wash solution. In this case, the volume of wash solution is determined by the flow rate and the time.

A filter 120 is provided within the first compartment 140 and rests on porous support 110, which provides mechanical support for and determines the location of filter 120. Filter 120 essentially spans the entire cross-section of the container. The active filter area of filter 120 need not occupy the entire cross-sectional area. However, the shape and structure of filter 120 should effectively fill the cross-sectional area such that solutions placed in compartment 140 have to pass through filter 120 in order to exit container 100 via second opening 150.

Generally, a pressure gradient is used to force solutions placed in compartment 140 to pass through filter 120. A positive pressure may be applied via first opening 130 to compartment 140. In other embodiments, a negative pressure may be applied via second opening 150 to draw the solution through filter 120. In some embodiments the apparatus may provide for both or either application of a positive pressure at the entrance side or a negative pressure at the exit side of the container. In some cases, the procedure for passing a liquid through the filter may include applying via first opening 130 a positive pressure, then a negative pressure, and then a positive pressure, wherein at least some of the liquid would be moved into, then back across, and then through the filter.

The amount of pressure necessary to drive solutions through the filter depends on many factors, such as pore size and void volume of the filter, the viscosity of the solution, and the desired residence time of the solution in the filter while it is being passed through. Those of skill in the art can readily determine useful operating ranges of the pressure depending on these factors. In some embodiments, the same operating pressure is used in all steps (e.g., adsorption, wash, elution). In some embodiments, pressure applied to each solution is tailored to the properties and/or the purpose of each solution. For example, a higher pressure differential may be used to pass higher viscosity solutions through the filter. And, the pressure differential can be used to adjust the time during which the solution contacts the filter. For example, the time for the adsorption step may be adjusted to allow sufficient time for the nucleic acid materials to adsorb to the solid phase. The time generally depends on the pore size, void volume, solution viscosity, size of nucleic acids, and the like, and can be readily optimized for a particular filter. The pressure, and thus the time for each wash can be readily optimized based on the yield of purified material obtained, in view of the timing requirements for the process, particularly when the process is automated. Typically, a pressure in the range of up to 300 kPa is used in the methods. In some embodiments, a solution may be allowed to pass through the filter under the force of gravity and capillary action without actively applying a pressure differential.

In some embodiments, the nucleic acid material is part of a solution, which may be, for example, a cellular lysate, a tissue lysate, a clinical sample processed to make available the relevant nucleic acid material, or other reaction solution. The solution further contains, in some embodiments, agents to foster the adsorption of nucleic acid material onto the solid phase, such as pH buffers, salts, denaturing agents, and/or chaotropes, as are well known in the art. Typically, the concentration of the nucleic acid material in the solution is 1 to 10000 copies/1 nL. Typically, the mass of nucleic acid material purified from the solution is 1 fg to 50 µg.

To adsorb the nucleic acid material onto the filter, the solution containing the nucleic acid material is added to first compartment 140, and then passed through the filter by applying a pressure gradient. Depending on the solution volume and the volume of first compartment 140, the process may need to be repeated one or more times to process all of the nucleic acid material. The flow rate of the sample solution is generally optimized to allow sufficient time for the nucleic acids to contact and bind to the filter material in order to maximize the adsorption of nucleic acids.

Once the nucleic acid material has been adsorbed, the filter is washed. The filter is washed with an immiscible fluid wash solution. The volume of wash solution is placed in first compartment 140, and a pressure gradient is applied to force the wash solution through the filter. The filter may be washed one or more times with the immiscible fluid wash solution.

In another embodiment, following adsorption, the filter is first washed with an alcohol-based wash solution and is then washed with an immiscible fluid wash solution. Any protocol using an alcohol wash step should be developed to ensure that alcohol is not present in the final eluate if the alcohol would inhibit or interfere with any subsequent processes involving the nucleic acids. For example, alcohol is recognized to be an inhibitor of reverse transcriptase and polymerase, and it may be preferable that the wash step between the alcohol wash and elution are sufficient to remove alcohol from the system. The volume of each wash type varies independently. In some embodiments, one or more batches may be needed to reach the desired volume of alcohol wash solution and immiscible fluid wash solution.

In another embodiment, following adsorption, the filter is first washed with an immiscible fluid wash solution, then with an alcohol-based wash solution, and then again with an immiscible fluid wash solution. The volume of each wash type varies independently. In some embodiments, one or more batches may be needed to reach the desired volume of wash solution.

After the wash step(s) have been completed, the nucleic acid material is eluted from the filter in an elution step. To elute the material, a volume of elution solution is added to first compartment 140, a pressure gradient is applied, and the eluted material is collected as it exits second opening 150. In some embodiments, the elution solution is permitted to contact the filter for several seconds, or tens of seconds, or a minute or more, such as five or ten minutes or more, before the pressure gradient is applied. In some embodiments the volume of elution solution used is about 20-60 µL. A larger volume may be used if a more dilute nucleic acid sample is acceptable. In some embodiments, for example as described in the examples, the elution solution volume is 30-50 µL. After the elution solution is passed through, an immiscible fluid push solution is added to first compartment 140 and passed through the filter to push through any residual elution solution, and collected. This use of an immiscible fluid push solution to push through the residual elution solution is useful in embodiments in which the amount of purified nucleic acid collected is to be maximized. For example, if the elution solution volume is 50 µL, and 10 µL of the eluate is sufficient for subsequent processing steps, then an immiscible fluid push solution may not be necessary, but if a larger volume, e.g., 25 µL, of the eluate is desired, then an immiscible fluid push solution may be beneficial.

In other embodiments of the elution step, to elute the material, a volume of elution solution is added to first compartment 140, then a volume of immiscible fluid push solution is layered on top of the elution solution, and then a pressure gradient is applied. The additional immiscible fluid push solution assists in forcing the aqueous-based elution solution through the filter and avoiding hold up of residual elution solution in the filter. The volume of the immiscible fluid push solution is 0.5 to 2-fold, preferably 1 to 1.5-fold the volume of the elution solution.

In one embodiment of the method for purifying DNA or RNA, a solution containing the DNA or RNA is passed through a glass microfiber filter to adsorb the DNA or RNA onto the microfibers, the filter is washed with a volume of an immiscible fluid wash solution (e.g., silicone oil), and then an elution solution (e.g., water) is passed through the filter, to elute the DNA or RNA into a collection tube. An immiscible fluid push solution is also used in the elution step according to any of the embodiments described above.

One embodiment of a DNA purification method comprises passing a solution containing the DNA through a glass microfiber filter to adsorb the DNA onto the microfibers, washing the filter with an alcohol-based wash solution (e.g., 50% ethanol in 10 mM Tris/100 mM NaCl solution), washing the filter with an immiscible fluid wash solution (e.g., silicone oil), and then eluting the adsorbed DNA with an elution solution (e.g., 10 mM Tris (pH 9.0) solution) into a collection tube. An immiscible fluid push solution is also used in the elution step according to any of the embodiments described above.

One embodiment of an RNA purification method comprises passing a solution containing the RNA through a glass microfiber filter to adsorb the RNA onto the microfibers, washing the filter with an immiscible fluid wash solution (e.g., silicone oil), washing the filter with an alcohol-based wash solution (e.g., 50% ethanol in 10 mM Tris/100 mM NaCl solution), washing the filter with an immiscible fluid wash solution (e.g., silicone oil), and then eluting the adsorbed RNA with an elution solution (e.g., RNase-free water) into a collection tube. An immiscible fluid push solution is also used in the elution step according to any of the embodiments described above.

The various solutions may be provided together, in whole or in part, as a kit, for the convenience of a user. The kit comprises an immiscible fluid wash solution, an elution solution and an immiscible fluid push solution. In another preferred embodiment, the kit comprises an immiscible fluid wash solution, an alcohol-based wash solution, an aqueous-based elution solution and an immiscible fluid push solution. In another preferred embodiment, the kit comprises an immiscible fluid wash solution, an alcohol-based wash solution, an elution solution, and an immiscible fluid push solution. In some embodiments, the same reagent can be used as both an immiscible fluid wash solution and an immiscible fluid push solution. The reagents may be provided in a volume sufficient for conducting 1 to 25 purification operations for a given system and type of container. Larger volumes are also contemplated, and may be desirable when the methods are performed by automated equipment.

In some embodiments a reagent may be provided as a concentrated solution, for example, as a 10x solution, to be diluted before use. In some embodiments, a reagent may be provided as the solid components, for example, as a lyophilized powder, to be reconstituted by adding the liquid component(s) for the solution before use. The liquid component for diluting or reconstituting a solution may also be provided in the kit. Typically, the liquid for dilution or reconstitution is water, which has a suitable purity for the application, as described above.

The kit may further comprise a document with instructions for using the reagents according to the methods of the invention.

In any of the above embodiments, the kit may further comprise a container fitted with a filter.

### Examples

### Example 1. Filter Device.

A filter device was prepared by fitting a Whatman glass microfiber filter, grade GF/B (Whatman, GE Healthcare Life Sciences, Piscataway, NJ) on top of a supporting ribbed structure in a cylindrical polypropylene cartridge. The Whatman filter was cut to be slightly larger than the internal diameter (6.0 mm) of the cartridge and press fitted into place against the supporting structure.

### Example 2. (reference) Purification of HIV RNA from Human Plasma.

In this example, ten immiscible fluids were tested for use as an immiscible wash solution for the purification of RNA from human plasma.

Human plasma negative for HIV was spiked with HIV-positive human plasma (Acrometrix OptiQual HIV-1 High Control) to provide human plasma samples containing 1000 HIV particles per 1 mL. A thioglycerol K4 lysis buffer was prepared immediately before use by adding 220 µL 1-thioglycerol (10 µL/mL) to 21.8 mL K4 lysis buffer (4 M guanidinium thiocyanate, 770 mM potassium acetate, 0.78% (w/v) N-lauryl sarcosine, 0.05 M Bis-Tris (pH 6.4)).

Samples (1.0 mL) were aliquoted into 5 mL tubes, and to this 1.8 mL thioglycerol-treated K4 lysis buffer was added, and the solution was mixed by vortexing. Carrier RNA was added (5 µL of 10 mg/mL of PolyA (Sigma Aldrich, St. Louis, MO)) and the solution was mixed by vortexing. After incubating the sample solution at 56°C for 10 minutes, absolute isopropanol (1.9 mL) was added and the solution was mixed by vortexing.

The solution was transferred in 700 µL portions to a filter device prepared according to Example 1. Each solution portion was passed through the filter by applying pressure (-40 kPa) to the liquid above the filter, and the process was repeated until all the sample was passed through the filter, to adsorb the RNA onto the filter.

Each filter was then washed with one of the immiscible fluid wash solutions shown in the Table below by adding 300 µL of the wash solution to the top of the filter and applying pressure above the solution to pass it through the filter.

**Table 1.**

| **Immiscible Wash Solution** | **Source** |
|---|---|
| Silicone fluid, 5 cSt | Clearco Products Co., Inc. (Bensalem, PA) |
| Silicone fluid, 50 cSt | Clearco Products Co., Inc. (Bensalem, PA) |
| Silicone fluid, 350 cSt | Clearco Products Co., Inc. (Bensalem, PA) |
| Mineral oil | Sigma Aldrich (St. Louis, MO) |
| Vapor Lock | Qiagen Inc., (Valencia, CA) |
| Olive oil, extra virgin | Bertolli (Unilever, Englewood Cliffs, NJ) |
| Soybean oil | Great Value (Walmart) |
| Fish oil (One A Day) | Bayer (Leverkusen, Ger.) |
| Baby oil (mom to mom®) | Safeway Inc. (Pleasanton, CA) |
| Peanut oil | Safeway Inc. (Pleasanton, CA) |

The filter was then washed with an alcohol-based wash solution. Aqueous ethanol solution (70% ethanol, 700 µL) was added on top of the filter and passed through the filter by applying pressure, and this wash process was repeated one more time.

The filter was washed again using the same immiscible fluid wash solution, in the same amount. After this second wash with the immiscible fluid wash solution, the cartridge was positioned over a new 0.5 mL centrifuge tube. An elution solution of water (50 µL) was added on top of the filter, allowed to stand in contact with the filter for 30 seconds, and then passed through the filter by pressurizing the space above the water to elute the adsorbed nucleic acids into the new tube.

Two types of controls were also prepared for comparative purposes. First, human plasma negative for HIV was processed as a sample without spiking the plasma with HIV (no target control), and second, HIV-positive samples were processed without including any washes with an immiscible wash solution (no immiscible wash control).

### Example 3. (reference) Analysis of HIV Samples by RT-PCR.

Primers for detecting HIV RNA were synthesized with the following sequences and used in a one-step RT-PCR reaction:
SEQ ID NO:1 (forward primer) 5'-AGTTGGAGACATCAAGCAGCCATGCAAAT
SEQ ID NO:2 (reverse primer) 5'-TGATATGTCAGTTCCCCTTGGTTCTCT.
The forward primer primes the formation of cDNA based on the HIV RNA target, and the forward and reverse primers operate as a primer pair to amplify a DNA product based on the cDNA strand. The expected DNA product is 155 bp.

A primer pre-mix solution was prepared, containing: 1x SensiFAST™ SYBR No-ROX One-Step mix (Bioline USA, Taunton, MA), 0.6 µM forward primer, 0.346 µM reverse primer, 10U/µL RNAse inhibitor, and 0.25 µL reverse transcriptase solution per 25 µL sample. RT-PCR reaction solutions were prepared by combining the primer pre-mix solution (15 µL) with sample (10 µL) purified according to Example 2. Each sample was analyzed by RT-PCR in duplicate. The theoretical amount of HIV in the RT-PCR analysis is 200 copies/sample.

The RT-PCR analysis was performed in a SmartCycler® SC1000-1 (Sunnyvale, CA) as follows.
RT stage: 45°C for 900 s, and 95°C for 120 s;
PCR stage: 40 cycles of 95°C for 5 s, 61°C for 5 s, and 72°C for 10 s.

A melting curve was measured during a temperature ramp of 0.2°C/sec from 60°C to 95°C after thermocycling was completed.

The RT-PCR product of each sample was also analyzed by electrophoresis using the 2100 Bioanalyzer (Agilent Technologies, Santa Clara, CA).

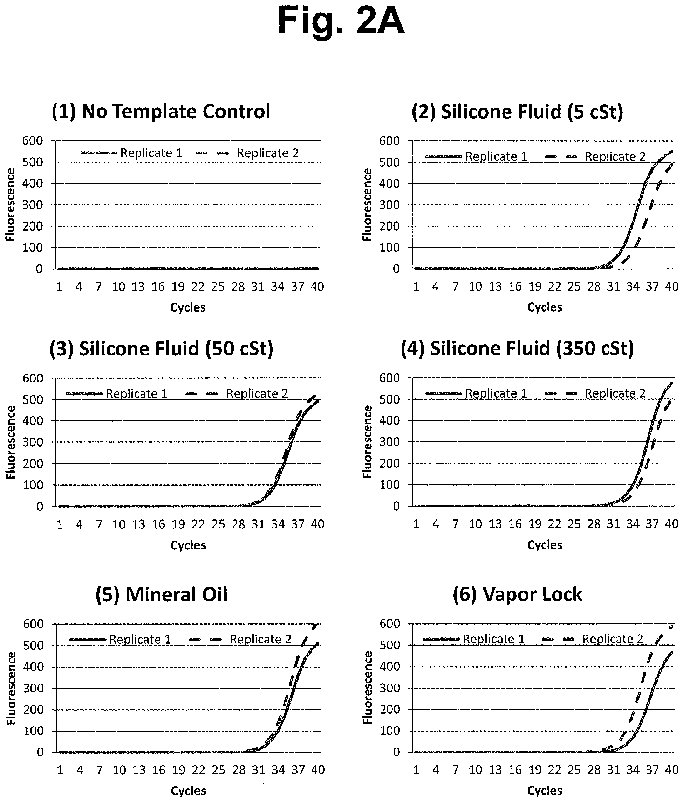

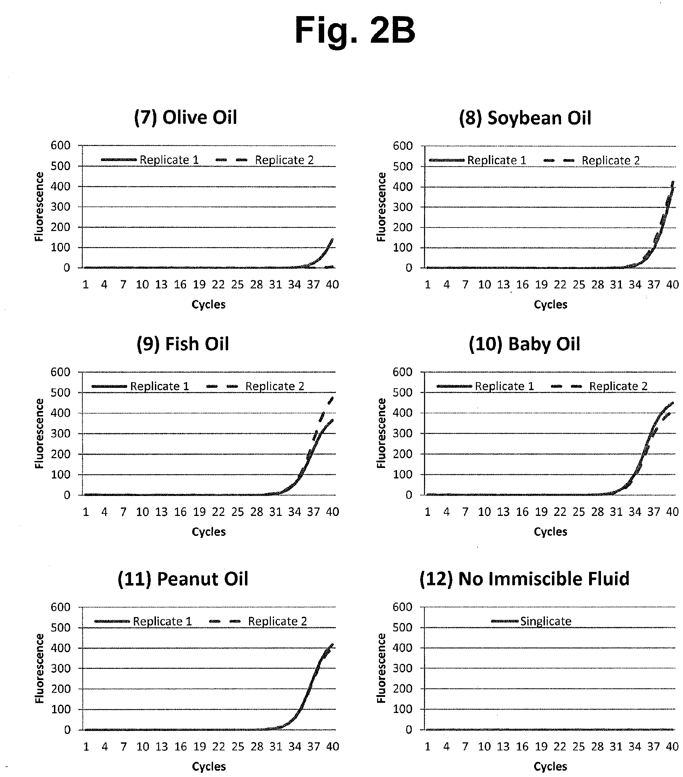

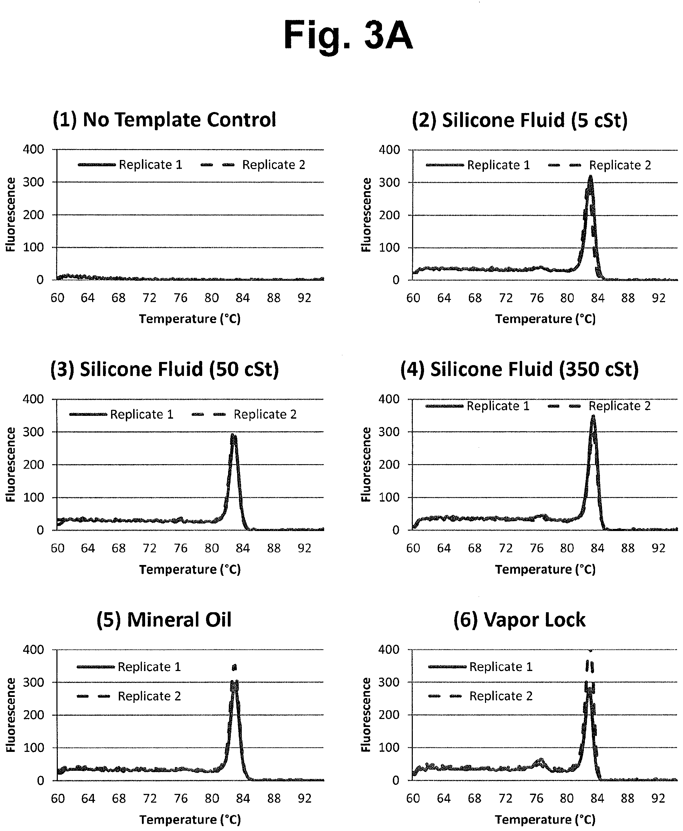

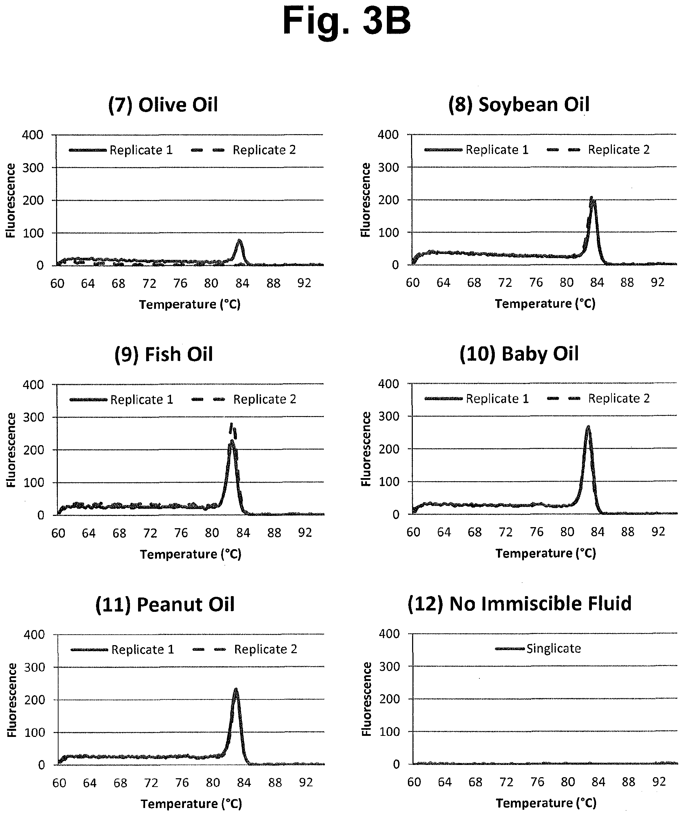

Fig. 2A-2B shows the growth curve and Fig. 3A-3B shows the melt peak graph measured in the SmartCycler® for the RT-PCR amplification of the ten samples (purified by washing with the ten different immiscible wash solutions) and the two controls.

As shown, the ten samples (analyzed in duplicate) each yielded the expected product. The cycle threshold (Ct) for each sample was observed to be about 32 (see Table 2) for eight of the samples. The similar Ct value for the various washes indicates that each immiscible wash solution was similarly efficient in removing PCR inhibitors, and the purification protocol as a whole was able to deliver similar amounts of purified RNA target. Two samples (soybean oil and olive oil) showed higher Ct values, suggesting that these fluids cause some inhibition. Whether this degree of inhibition is substantial depends on the needs and goals of the application. The "no immiscible wash" control was negative; no amplification product was observed, indicating that PCR inhibitors were not adequately removed by adsorbing the nucleic acids to the filter, washing with an alcohol wash solution, and eluting with water. The "no target" control was negative, indicating the absence of any contamination.

**Table 2.**

| **Sample** | **Cycle Threshold (Ct)** |
|---|---|
| (1) No target control | 0, 0 |
| (2) Wash with silicone fluid, 5 cSt | 30.5, 32.4 |
| (3) Wash with silicone fluid, 50 cSt | 31.6, 31.3 |
| (4) Wash with silicone fluid, 350 cSt | 32.1, 33.0 |
| (5) Wash with mineral oil | 32.0, 31.5 |
| (6) Wash with Vapor Lock | 32.6, 30.9 |
| (7) Wash with olive oil | 37.3, 0 |
| (8) Wash with soybean oil | 35.1, 34.6 |
| (9) Wash with fish oil | 33.1, 32.8 |
| (10) Wash with baby oil (mom to mom®) | 31.9, 32.2 |
| (11) Wash with peanut oil | 32.9, 33.0 |
| (12) No immiscible wash control | 0 |

The melt curves shown in Fig. 3A-3B show one peak at about 83-84°C for the ten samples, whereas the two controls have no peak, indicating that the samples contain a double-stranded amplification product whereas the control reactions do not.

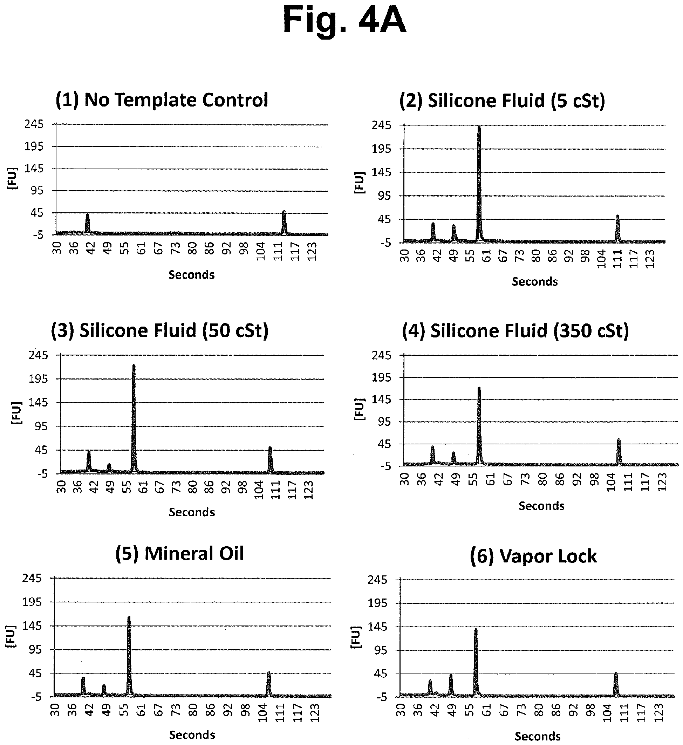

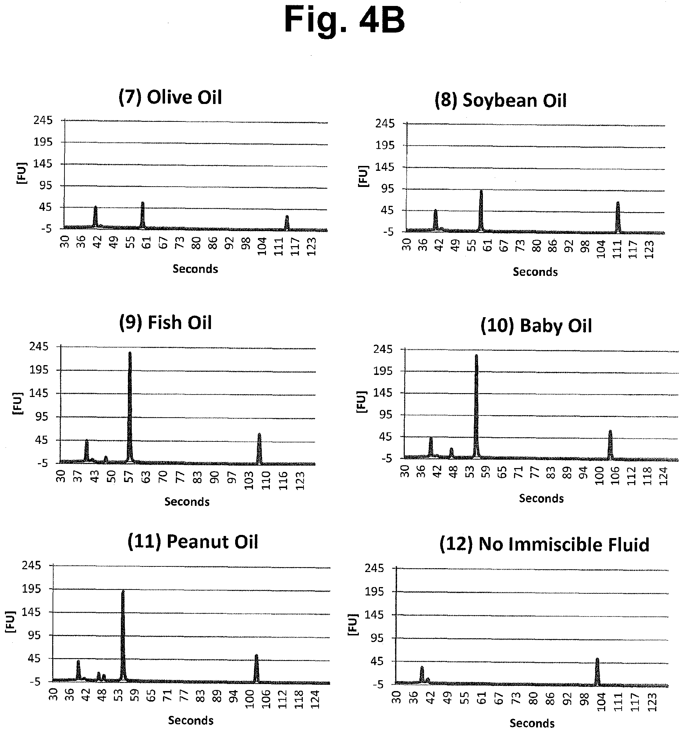

Fig. 4A-4B shows the electropherograms for the same samples and controls. The peak at about 58 sec corresponds to the 155 bp amplification product, and the peaks at about 41 sec and 110 sec are molecular size markers. The similarity of the results confirms that the RT-PCR reaction reproducibly yielded the expected amplification product with no other non-specific amplification reactions in the purified samples.

### Example 4 (reference). Purification of HBV DNA from Human Plasma.

In this example, four different volumes of immiscible fluid wash solution were compared for purifying DNA from human plasma in conjunction with reagents from a commercial kit, QuickGene DNA whole blood kit S (Fujifilm Corp.) for DNA sample preparation.

Human plasma negative for HBV was spiked with HBV-positive human plasma (Acrometrix OptiQual HBV High Positive Control) to provide human plasma samples containing 28,150 HBV particles per 1 mL.

QuickGene DB-S protease solution (EDB) (150 µL) was placed in the bottom of a 5 mL conical tube, and then the spiked HBV sample (1.0 mL) was aliquoted into the tube. QuickGene DB-S lysis buffer (LDB) (1.25 mL) was added to each tube, and the mixture was immediately mixed by pipette, vortexed, and incubated at 56°C for 2 minutes. Absolute ethanol (1.25 mL) was added and the solution was mixed by vortexing.

The solution was transferred in 600 µL portions to a filter device prepared according to Example 1. Each solution portion was passed through the filter by applying pressure (-40 kPa) to the liquid above the filter, and the process was repeated until all the sample was passed through the filter, to adsorb the DNA thereto.

Each filter with adsorbed DNA was then washed three times with QuickGene DB-S alcohol-based wash buffer (WDB) (750 µL). Next, each filter was washed once with an immiscible fluid wash solution, silicone fluid (5 cSt), by adding either 50 µL, 100 µL, 300 µL, or 750 µL of the wash solution to the top of the filter and applying pressure above the solution to pass it through the filter.

After this wash with the immiscible fluid wash solution, the cartridge was positioned over a clean 0.5 mL centrifuge tube. QuickGene DB-S elution buffer (50 µL) was added on top of the filter, allowed to stand in contact with the filter for 30 seconds, and then passed through the filter by pressurizing the space above the solution to elute the adsorbed nucleic acids into the clean tube.

Two types of controls were also prepared for comparative purposes. First, human plasma negative for HBV was processed as a sample without spiking the plasma with HBV (no target control), and second, HBV-positive samples were processed and adsorbed to the filter, but the filter was not washed with the immiscible fluid wash solution ("no immiscible fluid wash" control).

### Example 5. (reference) Analysis of HBV Samples by PCR.

Primers for detecting HBV DNA were synthesized with the following sequences and used in a PCR reaction:
SEQ ID NO:3 (forward primer) 5'-GACCACCAAATGCCCCTAT
SEQ ID NO:4 (reverse primer) 5'-TGAGATCTTCTGCGACG.
The forward and reverse primers operate as a primer pair to amplify a 132 bp sequence of the HBV DNA target.

A primer pre-mix solution was prepared, containing: 1x SensiFAST™ SYBR No-ROX Kit (Bioline USA, Taunton, MA), 0.4 µM forward primer, and 0.4 µM reverse primer per 25 µL sample. PCR reaction solutions were prepared by combining the primer pre-mix solution (15 µL) with sample (10 µL) purified according to Example 4. Each sample was analyzed by PCR in duplicate. The theoretical amount of HBV in the RT-PCR analysis is 5630 copies/sample.

The PCR analysis was performed in a SmartCycler® SC1000-1 (Cepheid, Sunnyvale, CA) as follows:
Initial denaturation: 95°C for 120 s;
PCR stage: 40 cycles of 95°C for 5 s, 57°C for 10 s, and 72°C for 15 s.

A melting curve was measured during a temperature ramp of 0.2°C/sec from 60°C to 98°C after thermocycling was completed.

The PCR product of each sample was also analyzed by electrophoresis using the 2100 Bioanalyzer (Agilent Technologies, Santa Clara, CA).

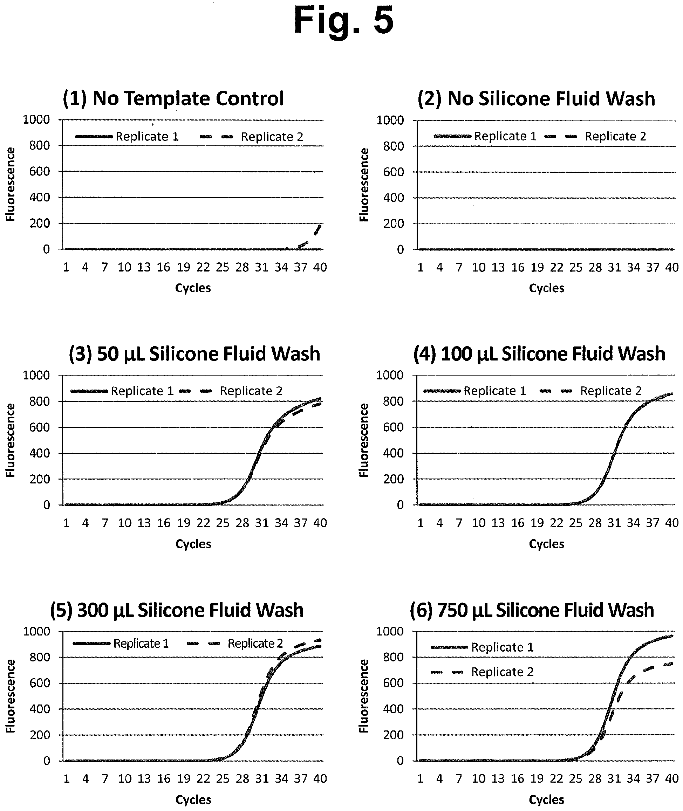

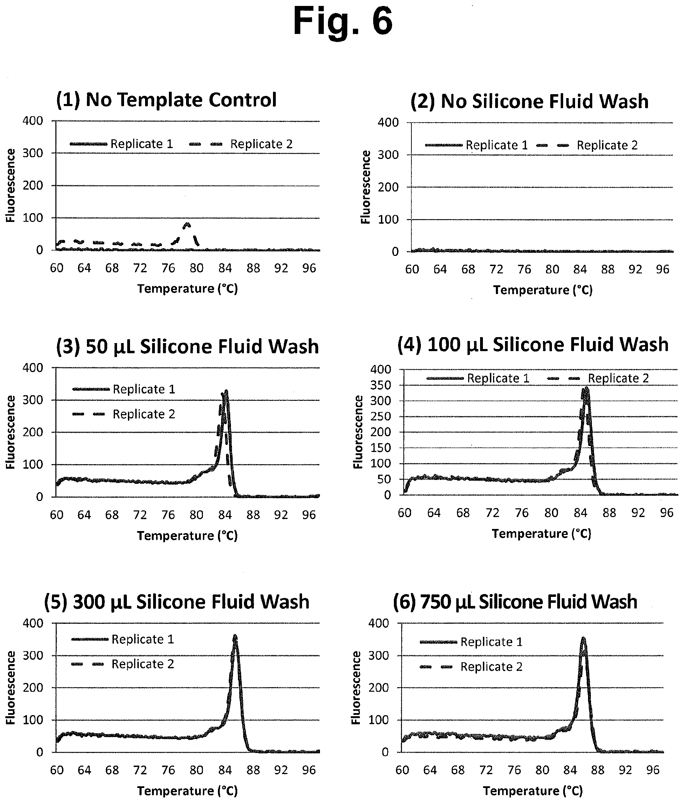

Fig. 5 shows the growth curve and Fig. 6 shows the melt peak graph measured in the SmartCycler® for the PCR amplification of the four samples (purified by washing with four different volumes of immiscible fluid wash solution) and the two controls.

As shown, the four samples (analyzed in duplicate) each yielded the expected product. The cycle threshold (Ct) for each sample was observed to be about 26 (see Table 2) for the four samples. The similar Ct value for the various washes indicates that each immiscible wash solution was similarly efficient in removing PCR inhibitors, and the purification protocol as a whole was able to deliver similar amounts of purified DNA target. The melting temperature for the amplification product shows a slight increase with increasing volume of immiscible fluid wash.

When the adsorbed DNA sample is not washed with the immiscible fluid wash solution, however, no amplification product is obtained. The "no immiscible fluid wash" control was negative, indicating that PCR inhibitors were not adequately removed by the standard protocol of adsorbing the nucleic acids to the filter, washing with the commercial wash solution, and then eluting with the elution buffer. One of the "no target" controls showed a small amount of a non-specific amplification product, but otherwise the negative controls were negative.

**Table 3.**

| **Sample** | **Cycle Threshold (Ct)** | **Melt Peak (°C)** |
|---|---|---|
| (1) No target control | 0,37 | --, 79.1 |
| (2) No immiscible fluid wash control | 0, 0 | --, -- |
| (3) Wash with 50 µL silicone fluid (5 cSt) | 25.7, 25.9 | 84.4, 84.1 |
| (4) Wash with 100 µL silicone fluid (5 cSt) | 26.3, 26.2 | 85.1, 84.8 |
| (5) Wash with 300 µL silicone fluid (5 cSt) | 25.7, 25.5 | 85.9, 85.8 |
| (6) Wash with 750 µL silicone fluid (5 cSt) | 25.7, 26.1 | 86.2, 86.3 |

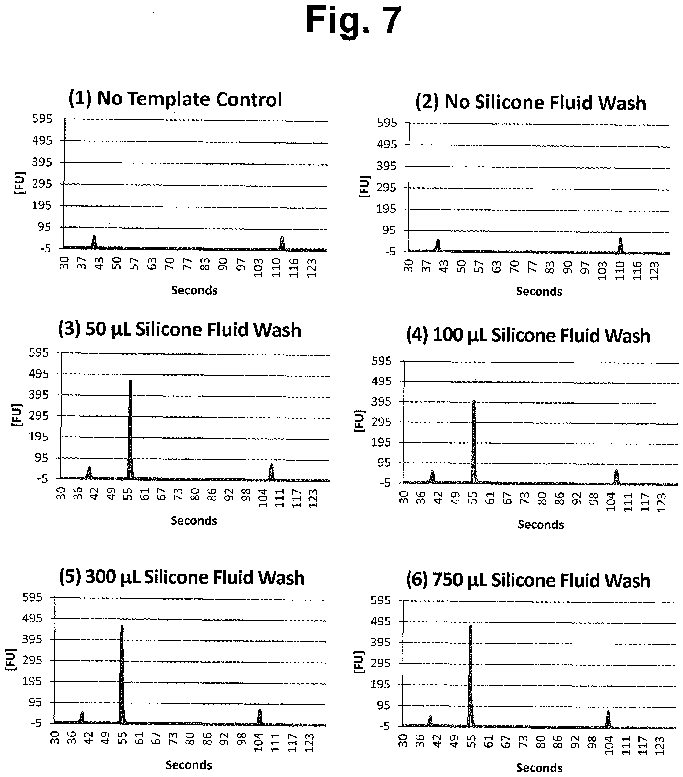

Fig. 7 shows the electropherograms for the same samples and controls. The peak at about 58 sec corresponds to the 132 bp amplification product. The similarity of the results confirms that the PCR reaction reproducibly yielded the expected amplification product with no non-specific amplification reactions in the purified samples.

### Example 6. Genomic DNA Collection Efficiency.

### (1) Extraction of Mycobacterium bovis BCG gDNA.

Colonies of *Mycobacterium bovis* BCG (Japanese Society for Bacteriology) incubated in 2% Ogawa medium (S) (Kyokuto Pharmaceutical Industries) for 28 days were collected, suspended in sterilized water, and autoclaved (121°C for 20 minutes). Next, the DNA was purified using Qiagen's DNA Genomic-tip extraction-purification kit. The copy number was determined by measuring the optical absorbance of the obtained purified gDNA.

### (2) Nucleic acid purification method by nucleic acid purification cartridge.

Samples containing *Mycobacterium bovis* BCG gDNA (copy number of 3 × 10³) and 2 µg of salmon sperm DNA (SIGMA) in 900 µL of 40% isopropyl alcohol aqueous solution containing 1.7 M guanidine hydrochloride (Wako Pure Chemical) were prepared. The sample was transferred to a filter device prepared according to Example 1, and passed through the filter (6 kPa) to adsorb the DNA onto the filter.

The filter was first washed by passing through the filter 700 µL of a first wash solution consisting of 50% ethanol aqueous solution containing 50 mM Tris-HCI (pH 8.0) (Wako Pure Chemical), 150 mM sodium chloride (Wako) and 1% Tween 80 (Wako).

The filter was then washed with 700 µL of a second wash solution consisting of 50 % ethanol aqueous solution containing 50 mM Tris-HCI (pH 8.0) (Wako Pure Chemical) and 150 mM sodium chloride (Wako Pure Chemical). Next, 300 µL of a first immiscible fluid wash solution consisting of silicone oil (viscosity: 5 cs) (Shin-Etsu Chemical) was passed through the filter by applying pressure (-25-100 kPa) above the wash solution.

To elute the DNA, first 30 µL of 10 mM Tris-HCI (pH 8.0) (Wako Pure Chemical) elution solution was added to the cartridge and allowed to contact the glass microfiber filter for one to five minutes, and then 30 µL of an immiscible fluid push solution consisting of silicone oil (viscosity: 50 cs, Shin-Etsu Chemical) was added on top of the elution buffer. The elution solution and immiscible fluid push solution were passed through the filter by applying pressure (∼25-100 kPa) above the solutions.

### (3) Volume Yield of Eluted Sample

The cartridge purification method described in section (2) of this Example was performed for eight samples, and the volume of sample collected by elution was measured. As a comparison, the purification method of section (2) was also performed omitting the immiscible fluid push solution. The results are in Table 4.

**Table 4.**

| **Method** | **Eluted Volume (µL)** | **Average Volume (µL)** |
|---|---|---|
| Method of Section (2) | 25 | 25.6 ± 0.6 |
| | 26 | |
| | 25 | |
| | 26 | |
| | 25 | |
| | 26 | |
| | 26 | |
| | 26 | |
| Comparative Method (without using an immiscible fluid push solution) | 5 | 7.7 ± 2.7 |
| | 10 | |
| | 8 | |

As shown in Table 4, the volume of eluent collected is low and varies (from 5 to 10 µL) when the immiscible fluid push solution is omitted from the protocol. As illustrated by the data, using only an aqueous-based elution solution as in the comparative method, the desired amount of eluent, at least 12.5 µL, to implement the quantitative measurement described in section (4) below was not obtained. Using the immiscible fluid wash solutions provides at least three benefits. The volume collected meets the volume requirements of subsequent analytical steps, the eluted volumes are more consistent, which aids in automation and data analysis, and the amount of nucleic acid in the initial sample can be more accurately determined because the eluted volume is more nearly quantitative.

### (4) Quantitative Analysis of Eluted Samples by Real-Time PCR.

Four samples (900 µL) containing 3 × 10³ copies of *Mycobacterium bovis* BCG gDNA were prepared and purified according to the protocol of sections (1) and (2) of this Example, and the amount of DNA in the eluent was measured by real-time PCR using the SmartCycler® II (Cepheid).

Primers for detecting the insertion sequence IS6110 in *M*. *bovis* were synthesized with the following sequences:
SEQ ID NO:5 (forward primer) 5'-TGGGTAGCAGACCTCACCTAT
SEQ ID NO:6 (reverse primer) 5'-AACGTCTTTCAGGTCGAGTACG
SEQ ID NO:7 (probe) 5' FAM-TGGCCATCGTGGAAGCGACCCGC-TAMRA
FAM is the fluorescent dye fluorescein, and TAMRA is the fluorescent dye tetramethylrhodamine. The forward and reverse primers operate as a primer pair to amplify a 192 bp sequence of the IS6110 target.

PCR reaction solutions were prepared with (final concentration): 1x Taq buffer (TaKaRa), 3 mM MgCl₂ (TaKaRa), 400 mM dNTP (TaKaRa), 1 unit of TaKaRa Ex HS polymerase, 500 nM forward primer, 500 nM reverse primer, and 280 nM of probe oligo.

The PCR analysis was performed in a SmartCycler® II (Cepheid, Sunnyvale, CA) as follows:
Initial denaturation: 97°C for 60 s;
PCR stage: 40 cycles of 97°C for 6 s, 61 °C for 6 s, and 72°C for 6 s.

The fluorescence generated by dye released from the probe oligo was measured for each of the four samples. The Ct values determined on the SmartCycler® II are shown in Table 5. The yield of gDNA obtained in the eluent in the purification protocol was also determined by calibrating the quantitative result against a control sample prepared with the same concentration of gDNA (3 x 10³ copies/900 µL) but not subjected to the purification protocol of section (3). The Ct values for duplicate control samples were 28.44 and 28.92. The average, Ct = 28.68, is taken to represent 100% yield. As demonstrated by the calculated yield of DNA, essentially all of the gDNA was adsorbed and then released in the purification process.

**Table 5.**

| **Sample** | **Ct Value** | **Yield of Eluted DNA** |
|---|---|---|
| 1 | 28.45 | 117% |
| 2 | 28.52 | 112% |
| 3 | 28.71 | 98% |
| 4 | 28.59 | 106% |
| Average | 28.6 | 108% |

## Claims

1. A method of purifying polynucleic acids, the method comprising:
(a) absorbing the polynucleic acids to a filter fitted in a container;
(b) washing the filter with a first immiscible fluid wash solution; and
(c) eluting the polynucleic acids from the filter using (i) an aqueous-based elution solution and (ii) an immiscible fluid push solution;
wherein a solution of purified polynucleic acids is obtained.

2. The method of claim 1, further comprising:
after step (a) and before step (b), washing the filter with an alcohol-based wash solution.

3. The method of claim 1, further comprising:
after step (a) and before step (b), washing the filter with a second immiscible fluid wash solution and then washing the filter with an alcohol-based wash solution.

4. The method of any of claims 1, 2, or 3, wherein the polynucleic acids are DNA and/or RNA.

5. The method of claim 1, wherein the filter is a glass microfiber filter.

6. The method of claim 2, wherein the first immiscible fluid wash solution comprises a fluid that (i) when contacted with water forms a substantially separate phase from the water; and (ii) has a specific gravity less than that of water.

7. The method of claim 6, wherein the specific gravity of the first immiscible fluid wash solution is less than that of the alcohol-based wash solution used in the method.

8. The method of claim 2, wherein the alcohol-based wash solution comprises ethanol and water.

9. The method of claim 8, wherein the alcohol-based wash solution further comprises a salt and/or a buffer.

10. The method of claim 8, wherein the alcohol-based wash solution further comprises a hydrophilic polymer.

11. The method of claim 10, wherein the hydrophilic polymer is a polyethylene glycol polymer.

12. The method of claim 1, wherein the aqueous-based elution solution comprises a buffer.

13. The method of claim 1, wherein the aqueous-based elution solution comprises DNase/RNase-free water.

## Patentansprüche

1. Verfahren zum Reinigen von Polynukleinsäuren, wobei das Verfahren umfasst:
(a) Absorbieren der Polynukleinsäuren zu einem Filter, der in einen Behälter eingepasst ist,
(b) Waschen des Filters mit einer ersten Waschlösung aus nicht mischbarem Fluid und
(c) Eluieren der Polynukleinsäuren aus dem Filter unter Verwendung (i) einer Elutionslösung auf Wasserbasis und (ii) einer Push-Lösung aus nicht mischbarem Fluid,
wobei eine Lösung von gereinigten Polynukleinsäuren erhalten wird.

2. Verfahren nach Anspruch 1, ferner umfassend:
nach Schritt (a) und vor Schritt (b), Waschen des Filters mit einer Waschlösung auf Alkoholbasis.

3. Verfahren nach Anspruch 1, ferner umfassend:
nach Schritt (a) und vor Schritt (b), Waschen des Filters mit einer zweiten Waschlösung aus nicht mischbarem Fluid und dann Waschen des Filters mit einer Waschlösung auf Alkoholbasis.

4. Verfahren nach einem der Ansprüche 1, 2 oder 3, wobei die Polynukleinsäuren DNA und/oder RNA sind.

5. Verfahren nach Anspruch 1, wobei der Filter ein Glasmikrofaserfilter ist.

6. Verfahren nach Anspruch 2, wobei die erste Waschlösung aus nicht mischbarem Fluid ein Fluid umfasst, das (i) bei Kontakt mit Wasser eine im Wesentlichen vom Wasser getrennte Phase bildet; und (ii) eine relative Dichte aufweist, die geringer ist als die von Wasser.

7. Verfahren nach Anspruch 6, wobei die relative Dichte der ersten Waschlösung aus nicht mischbarem Fluid geringer ist als die der im Verfahren verwendeten Waschlösung auf Alkoholbasis.

8. Verfahren nach Anspruch 2, wobei die Waschlösung auf Alkoholbasis Ethanol und Wasser umfasst.

9. Verfahren nach Anspruch 8, wobei die Waschlösung auf Alkoholbasis ferner ein Salz und/oder einen Puffer umfasst.

10. Verfahren nach Anspruch 8, wobei die Waschlösung auf Alkoholbasis ferner ein hydrophiles Polymer umfasst.

11. Verfahren nach Anspruch 10, wobei das hydrophile Polymer ein Polyethylenglycolpolymer ist.

12. Verfahren nach Anspruch 1, wobei die Elutionslösung auf Wasserbasis einen Puffer umfasst.

13. Verfahren nach Anspruch 1, wobei die Elutionslösung auf Wasserbasis DNase/RNase-freies Wasser umfasst.

## Revendications

1. Procédé de purification d'acides polynucléiques, le procédé comprenant :
(a) l'absorption des acides polynucléiques sur un filtre installé dans un récipient ;
(b) le lavage du filtre à l'aide d'une première solution de lavage de fluide non miscible ; et
(c) l'élution des acides polynucléiques à partir du filtre en utilisant (i) une solution d'élution à base aqueuse et (ii) une solution de fluide non miscible exerçant une poussée ;
dans lequel une solution d'acides polynucléiques purifiés est obtenue.

2. Procédé selon la revendication 1, comprenant en outre :
après l'étape (a) et avant l'étape (b), le lavage du filtre à l'aide d'une solution de lavage à base d'alcool.

3. Procédé selon la revendication 1, comprenant en outre :
après l'étape (a) et avant l'étape (b), le lavage du filtre à l'aide d'une seconde solution de lavage de fluide non miscible et ensuite le lavage du filtre à l'aide d'une solution de lavage à base d'alcool.

4. Procédé selon l'une quelconque des revendications 1, 2 ou 3, dans lequel les acides polynucléiques sont de l'ADN et/ou de l'ARN.

5. Procédé selon la revendication 1, dans lequel le filtre est un filtre à microfibres de verre.

6. Procédé selon la revendication 2, dans lequel la première solution de lavage de fluide non miscible comprend un fluide qui (i) lorsqu'il est mis en contact avec de l'eau forme une phase sensiblement séparée de l'eau ; et (ii) a une densité relative inférieure à celle de l'eau.

7. Procédé selon la revendication 6, dans lequel la densité relative de la première solution de lavage de fluide non miscible est inférieure à celle de la solution de lavage à base d'alcool utilisée dans le procédé.

8. Procédé selon la revendication 2, dans lequel la solution de lavage à base d'alcool comprend de l'éthanol et de l'eau.

9. Procédé selon la revendication 8, dans lequel la solution de lavage à base d'alcool comprend en outre un sel et/ou un tampon.

10. Procédé selon la revendication 8, dans lequel la solution de lavage à base d'alcool comprend en outre un polymère hydrophile.

11. Procédé selon la revendication 10, dans lequel le polymère hydrophile est un polymère de polyéthylène glycol.

12. Procédé selon la revendication 1, dans lequel la solution d'élution à base aqueuse comprend un tampon.

13. Procédé selon la revendication 1, dans lequel la solution d'élution à base aqueuse comprend de l'eau exempte de DNase/RNase.
